# EUROPEAN PATENT APPLICATION

(11) **EP 3 332 770 A1**
(43) Date of publication of application: **13.06.2018**
(21) Application number: 17205599.8
(22) Date of filing: 06.12.2017
(51) Int. Cl.: A61K 9/20, A61K 9/48, A61K 31/4439

(54) **PHARMACEUTICAL COMPOSITIONS OF DABIGATRAN**

(30) Priority: 07.12.2016 TR 201617984
(71) Applicant: Sanovel Ilac Sanayi ve Ticaret A.S., 34460 Istanbul (TR)
(72) Inventor: TÜRKYILMAZ, Ali, 34460 Istanbul (TR); PALANTÖKEN, Arzu, 34460 Istanbul (TR); GÜLKOK, Yildiz, 34460 Istanbul (TR); SÖZER BÜRKÜT, Gökce, 34460 Istanbul (TR)
(74) Representative: Sevinç, Erkan

(57) **Abstract**

The present invention relates to a pharmaceutical composition in the form of encapsulated mini tablets comprising dabigatran etexilate free base or pharmaceutically acceptable salts of dabigatran etexilate and at least one pharmaceutically acceptable excipient.

## Description

### Technical Field

The present invention relates to a pharmaceutical composition in the form of encapsulated mini tablets comprising dabigatran etexilate free base or pharmaceutically acceptable salts of dabigatran etexilate and at least one pharmaceutically acceptable excipient.

### Background of the Invention

Dabigatran etexilate (Formula 1), which is already known from WO 98/37075, is a direct thrombin inhibitor indicated to reduce the risk of stroke and systemic embolism in patients with non-valcular atrial fibrilation. Thrombin is a multifunctional enzyme which converts fibrinogen to fibrin, cross-linking fibrin monomers via activation of factor XIII and augmenting further thrombin production via the activation of factors V and VIII. It also activates platelets, generates anticoagulant activity via activation of protein C and initiates numerous cellular processes.

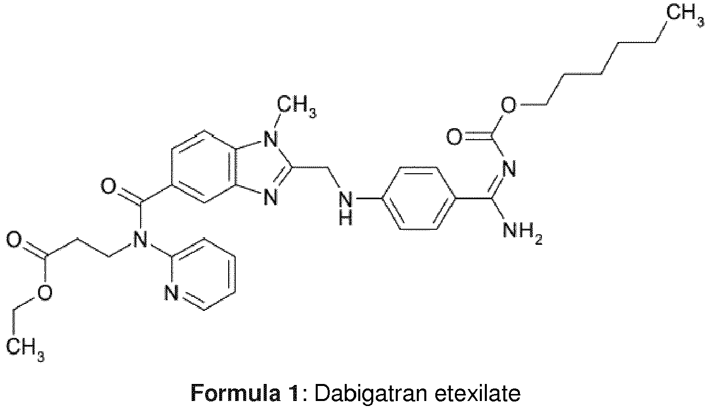

The methane sulphonic acid addition salt of dabigatran etexilate, which is commercially available under the trade name PRADAXA® ® immediate release capsule (in the strength of 75, 110, 150mg), is disclosed in EP1870100, wherein also disclosed, pellet formulation of dabigatran etexilate methanesulphonate. This composition is formulated with a core material consisting of organic acid and an active layer which encloses the core. Each PRADAXA® capsule contains the following inactive ingredients: acacia, dimethicone, hypromellose, hydroxypropylcellulose, tartaric acid, carragenan, potassium chloride, talc, titanium dioxide, and gelatin.

Apart from the methanesulfonate salt of dabigatran etexilate, other acid addition salts of the compound are provided in prior art. For exemple, WO2012/077136 is directed to the oxalate salt of dabigatran etexilate and besides, its hydrochloride salt is identified in EP1877395.

Also, it is known in the prior art that dabigatran etexilate is a weakly basic compound and therefore it has high solubility in acidic media.

Dabigatran etexilate have been associated with gastrointestinal disorders such as ulcers and gastrointestinal bleeding. In addition, the administration of dabigatran etexilate, may make some patients more susceptible to the ulcerogenic effects of ulcerogenic stimuli. It appears that a major factor contributing to the development of these gastrointestinal disorders is the presence of acid in the stomach and upper small intestine.

But also, in prior art, most of formulations are formulated with an acid excipient. This acidity may partly explain increased risk of dyspeptic symptoms and gastrointestinal bleeding.

Thus, there is still a need in the art to develop stable and bioavailable pharmaceutical formulations comprising dabigatran etexilate free base or pharmaceutically acceptable salts of dabigatran etexilate by an easy process. By this need, the formulation has been developed to overcome the solubility and stability problems of dabigatran etexilate in a safe manner disclosed above.

### Detailed description of the Invention

The main object of the present invention is to provide a pharmaceutical composition in the form of encapsulated mini tablets comprising dabigatran etexilate free base or pharmaceutically acceptable salts of dabigatran etexilate and at least one pharmaceutically acceptable excipient with safe and effective dissolution profile, an easy process and reduced adverse effects.

The term "mini tablet", as used herein, refers to small tablets with a diameter equal to or less than 4 mm that are typically filled into a capsule or further compressed into larger tablets. Thickness of this mini tablets equal to or less than 3 mm. The mini tablets have round shape and smooth surface to ease coating process.

Mini tablets have many advantages. Due to increased surface in relation to volume, the drug can be released more efficiently incase of mini-tablets. Some benefits of mini-tablets include excellent size uniformity, regular shape and a smooth surface. In mini tablets, smooth surface offers an excellent substrate for coating with polymeric systems. It can be concluded that pharmaceutical mini-tablets offer several advantages when compared to single unit dosage forms and are also good substitutes for granules and pellets. They have well defined size, shape, surface, low degree of porosity and high mechanical strength. Due to significant smaller dimensions of the mini tablets, when compared to normal tablets, they pass through the stomach at a more even rate. As a result, the concentration of the drug in the blood can be easily reproduced. It has been found that, dissolution and stability problems are overcome by using mini tablet form.

As used herein, the term "dabigatran etexilate free base" refers to dabigatran etexilate which is free from other forms of the active moiety, especially acid addition salts.

According to one embodiment, dabigatran etexilate free base or pharmaceutically acceptable salts of dabigatran etexilate is present in an amount of between 10.00 and 60.00 %, preferably between 20.00 and 50.00 % and more preferably it is 30.00 to 40.00 % by weight of total formulation.

According to another embodiment, dabigatran etexilate free base or pharmaceutically acceptable salts of dabigatran is present in an amount of between 30 to 350 mg, preferably 50 to 300 mg and more preferably it is 50 to 250 mg.

According to another embodiment of this present invention, wherein at least one pharmaceutically acceptable excipient is disintegrant. During the development study of the present invention, it has been observed that, dissolution and stability problems are overcome by using particularly at least two different disintegrants in a specific amount.

According to one embodiment, the pharmaceutical composition comprises preferably at least two different disintegrants.

According to another embodiment, the amount of total disintegrants is at least 40% by weight of total formulation. Preferably the amount of total disintegrants is between 40% and 60% by weight of total formulation. More preferably the amount of total disintegrants is between 40% and 50% by weight of total formulation.

Suitable disintegants are selected from a group comprising starch, sodium starch glycolate, microcrystalline cellulose, crospovidone (cross-linked polyvinil pyrrolidone), povidone, poloxomer, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, alginic acid, alginates, ion-exchange resins, magnesium aluminium silica, sodium dodesyl sulphate, poloxamer, sodium glycine carbonate, sodium lauryl sulphate or mixtures thereof.

In a preferred embodiment, the disintegrants are selected from a group comprising microcrystalline cellulose, pregelatinized starch and sodium starch glycolate.

According to this embodiment, microcrystalline cellulose is present in an amount of between 10.00% and 60.00%, preferably between 15.00% and 50.00%, more preferably between 25.00% and 40.00 by weight of total formulation, pregelatinized starch is present in an amount of between 1.00% and 30.00%, preferably between 5.00% and 20.00%, more preferably between 10.00% and 15.00% by weight of total formulation, sodium starch glycolate is present in an amount of between 0.1% and 8.00%, preferably between 0.25% and 4.00%, more preferably between 0.5% and 2.00% by weight of total formulation.

According to another embodiment of the present invention, the pharmaceutical composition further comprises lubricants, glidants, fillers or mixtures thereof.

Suitable lubricants are selected from a group comprising sodium stearyl fumarate, magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium lauryl sulphate or mixtures thereof.

In a preferred embodiment, the lubricant is selected from a group comprising magnesium stearate and sodium stearyl fumarate.

According to this embodiment, the lubricant is present an amount of between 0.1% and 10.00%, preferably 0.1% and 5.00% and more preferably 0.1% and 3.00% by weight of total formulation.

Suitable glidants are selected from a group comprising colloidal silicon dioxide, talc, aluminium silicate or mixtures thereof.

In a preferred embodiment, the glidant is colloidal silicon dioxide.

According to this embodiment, the glidant is present an amount of between 0.1% and 10.00%, 0.5% and 5.00% and more preferably 0.9% and 3.00% by weight of total formulation.

Suitable fillers are selected from a group comprising mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, polyols, dextrose, maltitol or mixtures thereof.

In a preferred embodiment, the filler is lactose.

According to this embodiment, the filler is present an amount of between 5.00% and 50.00%, 10.00% and 40.00% and more preferably 15.00% and 30.00% by weight of total formulation.

Suitable salts of dabigatran etexilate are selected from a group comprising mesylate, maleate, malonate, citrate, tosylate, esylate, tartrate, oxalate, camphor sulfonate.

Preferably suitable salts of dabigatran etexilate are selected from maleate, malonate, citrate, tosylate, esylate, tartrate, oxalate, camphor sulfonate.

In one embodiment, the pharmaceutical composition in the form encapsulated mini tablets comprises:
10.00 to 60.00 % dabigatran etexilate free base or pharmaceutically acceptable salts of dabigatran etexilate
10.00 to 60.00 % microcrystalline cellulose
5.00 to 50.00 % lactose
0.1 to 10.00 % colloidal silicon dioxide
1.0 to 30.00 % pregelatinized starch
0.1 to 8.00 % sodium starch glycolate
0.1 to 10.00 % magnesium stearate

In one embodiment, the pharmaceutical composition in the form encapsulated mini tablets comprises:
10.00 to 60.00 % dabigatran etexilate free base or pharmaceutically acceptable salts of dabigatran etexilate
10.00 to 60.00 % microcrystalline cellulose
5.00 to 50.00 % lactose
0.1 to 10.00 % colloidal silicon dioxide
1.0 to 30.00 % pregelatinized starch
0.1 to 10.00 % sodium stearyl fumarate

### Example 1: Capsule containing mini tablets

| **Ingredients** | **(%) amount (w/w)** |
|---|---|
| Dabigatran etexilate free base | 10-60 |
| Microcrystalline celluose | 10-60 |
| Lactose | 5-50 |
| Colloidal silicon dioxide | 0.1-10 |
| Starch, pregelatinize | 1-30 |
| Sodium starch glycolate | 0.1-8 |
| Magnesium stearate | 0.1-10 |
| **Total** | 100 |

The pharmaceutical compositions mentioned above are prepared by following these steps:
- Sieving and mixing dabigatran etexilate free base, sodium starch glycolate, microcrystalline cellulose, colloidal silicon dioxide, pregelatinized starch and lactose monohydrate
- Adding magnesium stearate to the powder mixture and mixing for 1-2 more minutes
- Compressing the powder mixture into mini tablets
- Filling the mini tablets into HPMC capsules.

### Example 2: Capsule containing mini tablets

| **Ingredients** | **(%) amount (w/w)** |
|---|---|
| Dabigatran etexilate free base | 30-40 |
| Microcrystalline celluose | 25-40 |
| Lactose | 15-30 |
| Colloidal silicon dioxide | 0.9-3 |
| Starch, pregelatinize | 10-15 |
| Sodium starch glycolate | 0.5-2 |
| Magnesium stearate | 0.1-2 |
| **Total** | 100.00 |

The pharmaceutical compositions mentioned above are prepared by following these steps:
- Sieving and mixing dabigatran etexilate free base, microcrystalline cellulose, lactose, colloidal silicon dioxide, pregelatinized starch, sodium starch glycolate.
- Granulating the powder mixture with water and drying in an oven at 50°C.
- Adding magnesium stearate and mixing 1-2 more minutes.
- Compressing the powder mixture into mini tablets
- Filling the mini tablets into HPMC capsules.

### Example 3: Capsule containing mini tablets

| **Ingredients** | **(%) amount (w/w)** |
|---|---|
| Dabigatran etexilate free base | 30-40 |
| Microcrystalline celluose | 25-35 |
| Lactose | 15-25 |
| Colloidal silicon dioxide | 0.9-3 |
| Starch, pregelatinize | 10-15 |
| Sodium stearyl fumarate | 0.1-3 |
| **Total** | 100 |

The pharmaceutical compositions mentioned above are prepared by following these steps:
- Sieving and mixing dabigatran etexilate free base, microcrystalline cellulose, lactose, colloidal silicon dioxide, pregelatinized starch, and half of sodium stearyl fumarate.
- Passing the powder mixture through the compactor or slugging
- Sieving the powder which passed through the compactor or grinding the slugs and sieving
- Adding the other half of sodium stearyl fumarate to the mixture and mixing for 1-2 more minutes
- Compressing the powder mixture into mini tablets
- Filling the mini tablets into HPMC capsules.

## Claims

1. A pharmaceutical composition in the form of encapsulated mini tablets comprising dabigatran etexilate free base or pharmaceutically acceptable salts of dabigatran etexilate and at least one pharmaceutically acceptable excipient.

2. The pharmaceutical composition according to claim 1, wherein the composition comprising dabigatran etexilate free base or pharmaceutically acceptable salts of dabigatran etexilate in an amount of 10.00% to 60.00% by weight of total formulation.

3. The pharmaceutical composition according to claim 1, wherein the amount of dabigatran etexilate free base or pharmaceutically acceptable salts of dabigatran etexilate is between 30 to 350 mg.

4. The pharmaceutical composition according to claim 1, wherein at least one pharmaceutically acceptable excipient is disintegrant.

5. The pharmaceutical composition according to claim 4, wherein the composition comprising preferably at least two different disintegrants.

6. The pharmaceutical composition according to claim 5, wherein the amount of total disintegrants is at least 40% by weight of total formulation.

7. The pharmaceutical composition according to claim 4, wherein the disintegrants are selected from a group comprising starch, sodium starch glycolate, microcrystalline cellulose, crospovidone (cross-linked polyvinil pyrrolidone), povidone, poloxomer, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, alginic acid, alginates, ion-exchange resins, magnesium aluminium silica, sodium dodesyl sulphate, poloxamer, sodium glycine carbonate, sodium lauryl sulphate or mixtures thereof, preferably the disintegrants are microcrystalline cellulose, sodium starch glycolate and pregelatinized starch.

8. The pharmaceutical composition according to claim 1, further comprising lubricants, glidants, fillers or mixtures thereof.

9. The pharmaceutical composition according to any of the preceding claims, comprising:
10.00 to 60.00 % dabigatran etexilate free base or pharmaceutically acceptable salts of dabigatran etexilate
10.00 to 60.00 % microcrystalline cellulose
5.00 to 50.00 % lactose
0.1 to 10.00 % colloidal silicon dioxide
1.0 to 30.00 % pregelatinized starch
0.1 to 8.00 % sodium starch glycolate
0.1 to 10.00 % magnesium stearate

10. The pharmaceutical composition according to any of the preceding claims, comprising:
10.00 to 60.00 % dabigatran etexilate free base or pharmaceutically acceptable salts of dabigatran etexilate
10.00 to 60.00 % microcrystalline cellulose
5.00 to 50.00 % lactose
0.1 to 10.00 % colloidal silicon dioxide
1.0 to 30.00 % pregelatinized starch
0.1 to 10.00 % sodium stearyl fumarate

11. The process for preparation of the pharmaceutical composition according to claim 9, wherein the process comprising the following steps:
a. Sieving and mixing dabigatran etexilate free base, sodium starch glycolate, microcrystalline cellulose, colloidal silicon dioxide, pregelatinized starch and lactose monohydrate
b. Adding magnesium stearate to the powder mixture and mixing for 1-2 more minutes
c. Compressing the powder mixture into mini tablets
d. Filing the mini tablets into HPMC capsules.

12. The process for preparation of the pharmaceutical composition according to claim 9, wherein the process comprising the following steps:
a. Sieving and mixing dabigatran etexilate free base, microcrystalline cellulose, lactose, colloidal silicon dioxide, pregelatinized starch, sodium starch glycolate.
b. Granulating the powder mixture with water and drying in an oven at 50°C.
c. Adding magnesium stearate and mixing 1-2 more minutes.
d. Compressing the powder mixture into mini tablets
e. Filling the mini tablets into HPMC capsules.

13. The process for preparation of the pharmaceutical composition according to claim 10, wherein the process comprising the following steps:
a. Sieving and mixing dabigatran etexilate free base, microcrystalline cellulose, lactose, colloidal silicon dioxide, pregelatinized starch, and half of sodium stearyl fumarate.
b. Passing the powder mixture through the compactor or slugging
c. Sieving the powder which passed through the compactor or grinding the slugs and sieving
d. Adding the other half of sodium stearyl fumarate to the mixture and mixing for 1-2 more minutes
e. Compressing the powder mixture into mini tablets
f. Filling the mini tablets into HPMC capsules.
